# EUROPÄISCHE PATENTANMELDUNG

(11) **EP 1 250 906 A2**
(43) Veröffentlichungstag der Anmeldung: **23.10.2002**
(21) Anmeldenummer: 02008071.9
(22) Anmeldetag: 11.04.2002
(51) Int. Cl.: A61K 7/00, A61K 7/06, A61K 7/48

(54) **Mittel zur Behandlung der Haare und/oder der Haut**

(30) Priorität: 20.04.2001 DE 10119683
(71) Anmelder: Hans Schwarzkopf GmbH & Co. KG, 22763 Hamburg (DE)
(72) Erfinder: Jung, Lars, 22309 Hamburg (DE); Goddinger, Dieter, Dr., 25336 Klein Nordende (DE); Seidel, Winfried, Dr., 25451 Quickborn (DE)
(74) Vertreter: Foitzik, Joachim Kurt

(57) **Zusammenfassung**

Wäßrige kosmetische Mittel zur Behandlung der Haare und/oder Haut, die neben üblichen kosmetischen Bestandteilen mindestens einen Wirkstoff (W) enthalten, der ein Addukt einer Glyceridkomponente (G), ausgewählt aus Mono-, Di- oder Triglyceriden, die als Säurekomponente mindestens eine einfach oder mehrfach ungesättigte Hydroxycarbonsäure mit 10-30 Kohlenstoffatomen aufweisen, mit einem cyclischen Carbonsäureanhydrid (CA) darstellt, enthalten, zeichnen sich durch hervorragende, pflegende, restrukturierende, feuchthaltende und rückfettende Eigenschaften aus.

## Beschreibung

Die Erfindung betrifft wäßrige kosmetische Mittel zur Behandlung der Haare und/oder der Haut mit einem speziellen Addukt aus einem Glycerid und einem cyclischen Carbonsäureanhydrid als Wirkstoff sowie die Verwendung dieser Mittel zur Behandlung von Haaren und/oder Haut.

Die Reinigung und Pflege von Haut und Haaren sowie die dekorative Gestaltung der Frisur sind wichtige Bestandteile der menschlichen Körperpflege. Entsprechend groß sind die Bemühungen, sowohl dem Friseur als auch dem Endverbraucher in jeder Hinsicht optimierte Produkte zur Verfügung zu stellen. Ein Schwerpunkt der Entwicklungsarbeit liegt dabei bei den eigentlichen Produkteigenschaften, sei es die Reinigungswirkung eines Shampoos, eines Duschbades oder einer Flüssigseife, die Pflegewirkung einer Haarkur oder Hautcreme, die Färbeeigenschaften eines Färbemittels oder einer Tönung, die Qualität einer Dauerwelle oder der Frisurenhalt eines Festigers oder Haarsprays. Ein besonderer Schwerpunkt liegt auf der Suche nach Substanzen, die in einer möglichst großen Anzahl unterschiedlicher Produkttypen pflegende Eigenschaften entfalten sowie dem Anwender ein positives Erlebnis, z. B. glänzende, geschmeidige Haare oder ein angenehmes, nicht trockenes Hautgefühl, vermitteln.

Es wurde nun überraschenderweise gefunden, daß spezielle Addukte aus einem Glycerid und einem cyclischen Carbonsäureanhydrid, insbesondere mit bestimmten weiteren Wirkstoffen, besonders hervorragende pflegende, restrukturierende, feuchthaltende und rückfettende Eigenschaften entfalten.
Gegenstand der Erfindung sind daher wäßrige kosmetische Mittel zur Behandlung der Haare und/oder der Haut, dadurch gekennzeichnet, daß sie neben üblichen kosmetischen Bestandteilen mindestens einen Wirkstoff (W) enthalten, der ein Addukt einer Glyceridkomponente (G), ausgewählt aus Mono-, Di- oder Triglyceriden, die als Säurekomponente mindestens eine einfach oder mehrfach ungesättigte Hydroxycarbonsäure mit 10-30 Kohlenstoffatomen aufweisen, mit einem cyclischen Carbonsäureanhydrid (CA) darstellt.

Die erfindungsgemäß eingesetzten Wirkstoffe (W) sind Addukte, die aus einer Glyceridkomponente (G) und einem Carbonsäureanhydrid (CA) bestehen.

Bei der Glyceridkomponente (G) kann es sich um ein Mono-, ein Di- oder ein Triglycerid handeln. Erfindungsgemäß bevorzugt handelt es sich bei der Glyceridkomponente (G) um ein Triglycerid. Weiterhin enthält die Glyceridkomponente als Säurekomponente mindestens eine einfach oder mehrfach ungesättigte Hydroxycarbonsäure mit 10-30 Kohlenstoffatomen. Unter Hydroxycarbonsäuren werden erfindungsgemäß sowohl Mono- als auch Di-, Tri- und Polyhydroxycarbonsäuren verstanden. Erfindungsgemäß bevorzugte Säurekomponenten sind einfach ungesättigte Monohydroxycarbonsäuren. Die Ricinolsäure ist eine erfindungsgemäß besonders bevorzugte Säurekomponente.

Als erfindungsgemäß besonders geeignete Glyceridkomponente (G) hat sich das Triglycerid der Ricinolsäure erwiesen.

Das cyclische Carbonsäureanhydrid (CA) wird erfindungsgemäß bevorzugt ausgewählt aus der Gruppe, die gebildet wird von Bernsteinsäureanhydrid und Maleinsäureanhydrid. Maleinsäureanhydrid ist ein erfindungsgemäß besonders bevorzugtes cyclisches Carbonsäureanhydrid (CA).

Einen erfindungsgemäß hervorragend geeigneten Wirkstoff (W) stellt das 1:1-Addukt aus Ricinusöl und Maleinsäureanhydrid dar, das als Handelsprodukt unter der Bezeichnung Ceraphyl®RMT (ISP) erhältlich ist. Die Herstellung dieses Addukts wird in der Offenlegungsschrift WO-A1-01/00148, auf deren Offenbarung ausdrücklich Bezug genommen wird, beschrieben.

Die Wirkstoffe (W) sind in den erfindungsgemäßen Mitteln in der Regel in Mengen von 0,2-10 Gew.-%, insbesondere in Mengen von 0,5-5 Gew.-%, und besonders bevorzugt in Mengen von 1-3 Gew.-%, jeweils bezogen auf das gesamte Mittel, enthalten.

Die Wirkungen der erfindungsgemäß eingesetzten Wirkstoffe (W) werden durch Kombination mit bestimmten weiteren Wirkstoffen in synergistischer Weise noch deutlich verstärkt.

Gemäß einer ersten bevorzugten Ausführungsform enthalten die erfindungsgemäßen Mittel weiterhin mindestens eine Vitaminkomponente, ausgewählt aus der Gruppe, die die Vitamine, Pro-Vitamine und Vitaminvorstufen der Gruppen A, B, C, F und H sowie deren Derivate bilden.

Zur Gruppe der als **Vitamin A** bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die erfindungsgemäßen Mittel enthalten die Vitamin A-Komponente bevorzugt in Mengen von 0,05-1 Gew.-%, bezogen auf die gesamte Zubereitung.

Zur **Vitamin B-Gruppe** oder zum Vitamin B-Komplex gehören u. a.
- Vitamin B₁ (Thiamin)
- Vitamin B₂ (Riboflavin)
- Vitamin B₃. Unter dieser Bezeichnung werden häufig die Verbindungen Nicotinsäure und Nicotinsäureamid (Niacinamid) gerührt. Erfindungsgemäß bevorzugt ist das Nicotinsäureamid, das in den erfindungsgemäßen Mitteln bevorzugt in Mengen von 0,05 bis 1 Gew.-%, bezogen auf das gesamte Mittel, enthalten ist.
- Vitamin B₅ (Pantothensäure und Panthenol). Im Rahmen dieser Gruppe wird bevorzugt das Panthenol eingesetzt. Erfindungsgemäß einsetzbare Derivate des Panthenols sind insbesondere die Ester und Ether des Panthenols sowie kationisch derivatisierte Panthenole und Vorstufen des Panthenols, wie z. B. Panthlacton. Einzelne Vertreter sind beispielsweise das Panthenoltriacetat, der Panthenolmonoethylether und dessen Monoacetat sowie die in der WO 92/13829 offenbarten kationischen Panthenolderivate. Die genannten Verbindungen des Vitamin B₅-Typs sind in den erfindungsgemäßen Mitteln bevorzugt in Mengen von 0,05 - 10 Gew.-%, bezogen auf das gesamte Mittel, enthalten. Mengen von 0,1 - 5 Gew.-% sind besonders bevorzugt.
- Vitamin B₆ (Pyridoxin sowie Pyridoxamin und Pyridoxal).

**Vitamin C** (Ascorbinsäure). Vitamin C wird in den erfindungsgemäßen Mitteln bevorzugt in Mengen von 0,1 bis 3 Gew.-%, bezogen auf das gesamte Mittel eingesetzt. Die Verwendung in Form des Palmitinsäureesters kann bevorzugt sein. Die Verwendung in Kombination mit Tocopherolen kann ebenfalls bevorzugt sein.

**Vitamin F.** Unter dem Begriff "Vitamin F" werden üblicherweise essentielle Fettsäuren, insbesondere Linolsäure, Linolensäure und Arachidonsäure, verstanden.

**Vitamin H.** Als Vitamin H wird die Verbindung (3a*S*,4*S*, 6a*R*)-2-Oxohexahydrothienol[3,4-*d*]-imidazol-4-valeriansäure bezeichnet, für die sich aber zwischenzeitlich der Trivialname Biotin durchgesetzt hat. Biotin ist in den erfindungsgemäßen Mitteln bevorzugt in Mengen von 0,0001 bis 0,1 Gew.-%, insbesondere in Mengen von 0,001 bis 0,01 Gew.-% enthalten.

Bevorzugt enthalten die erfindungsgemäßen Mittel Vitamine, Provitamine und Vitaminvorstufen aus den Gruppen A, B und H.

Panthenol und seine Derivate sowie Nicotinsäureamid als Vertreter der Vitamin B-Gruppe sowie Biotin sind besonders bevorzugte Vitaminkomponenten.

Gemäß einer zweiten bevorzugten Ausrührungsform enthalten die erfindungsgemäßen Mittel weiterhin mindestens ein Verdickungsmittel, ausgewählt aus der Gruppe, die alkoxylierte Fettalkohole, Zuckerderivate, gegebenenfalls vernetzte Polymere der Acrylsäure und der Methacrylsäure, alkoxylierte Methylglucosidester, alkoxylierte Propylenglykolester, Fettsäurepartialglyceridpolyglykolester und Fettsäurepartialglyceridpolyglykolether und deren Gemische umfaßt.

Alkoxylierte, insbesondere ethoxilierte Fettalkohole, insbesondere solche mit eingeschränkter Homologenverteilung sind beispielsweise als Handelsprodukt unter der Bezeichnung Arlypon®F (Cognis) im Handel erhältlich.

Bevorzugte Verdickungsmittel von Typ der Zuckerderivate sind beispielsweise
- Celluloseether, wie beispielsweise Hydroxyethylcellulose, Methylhydroxypropylcellulose, Methylhydroxyethylcellulose, Methylcellulose und Carboxymethylcellulose, die beispielsweise unter den Warenzeichen Natrosol®, Culminal® und Tylose® im Handel erhältlich sind,
- Xanthangumme, wie sie beispielsweise unter dem Warenzeichen Keltrol® im Handel erhältlich sind,
- Guar und Guar-Derivate, wie sie beispielsweise unter den Warenzeichen Cosmedia Guar® und Jaguar® im Handel erhältlich sind.

Als vernetzte Polymere der Acrylsäure oder Methacrylsäure sind erfindungsgemäß insbesondere die unter dem Warenzeichen Carbopol® erhältlichen Produkte, insbesondere Carbopol® 940, ETD 2001 und ETD 2020, verwendbar.

Ein Beispiel für einen erfindungsgemäß einsetzbaren alkoxylierten Methylglucosidester ist das Handelsprodukt Glucamate® DOE 120, ein Methylglucosiddioleat + 120 Ethylenoxid.

Ein Beispiel für einen erfindungsgemäß einsetzbaren alkoxylierten Propylenglykolester ist das Handelsprodukt Antil® 141, ein Poly(55)oxyethylen-propylenglykoldioleat.

Das Handelsprodukt Rewoderm®LIS-80 ist ein Beispiel für Gemische aus Fettsäurepartialglyceridpolyglykolethern und-estern.

Die genannten Verdickungsmittel können selbstverständlich auch in Kombination mit weiteren Verdickungsmitteln eingesetzt werden.

Die genannten Verdickungsmittel sind in den erfindungsgemäßen Mitteln bevorzugt in Mengen von 0,1-5 Gew.-%, insbesondere in Mengen von 0,5 3 Gew.-%, jeweils bezogen auf das gesamte Mittel, enthalten.

Gemäß einer dritten bevorzugten Ausführungsform enthalten die erfindungsgemäßen Mittel weiterhin mindestens ein kationisches oder pseudokationisches Tensid.

Beispiele für die in den erfindungsgemäßen Mitteln verwendbaren kationischen Tenside sind insbesondere quartäre Ammoniumverbindungen. Bevorzugt sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid Palmitylamidopropyltrimethylammoniumchlorid und Tricetylmethylammoniumchlorid.

Eine erfindungsgemäß bevorzugte Gruppe von kationischen Tensiden stellen die sogenannten Esterquats dar.

Bei diesen Esterquats handelt es sich um bekannte Stoffe, die man nach den einschlägigen Methoden der präparativen organischen Chemie erhalten kann. In diesem Zusammenhang sei auf die Internationale Patentanmeldung WO 91/01295 verwiesen, nach der man Triethanoiamin in Gegenwart von unterphosphoriger Säure mit Fettsäuren partiell verestert, Luft durchleitet und anschließend mit Dimethylsulfat oder Ethylenoxid quaterniert. Stellvertretend für den umfangreichen Stand der Technik sei an dieser Stelle auf die Druckschriften EP-A2 0 239 910, EP-A2 0 293 955, EP-A2 0 295 739 und EP-A2 0 309 052 sowie die. Übersichten von R.Puchta et al. in *Tens.Surf.Det.*, 30, 186 (1993), M.Brock in *Tens. Surf. Det.*, 30, 394 (1993) und R.Lagerman et al. in *J.Am.Oil.Chem.Soc*., 71, 97 (1994) verwiesen.

Esterquats, die quaternierte Fettsäuretriethanolaminestersalze darstellen, haben die allgemeine Formel (I), 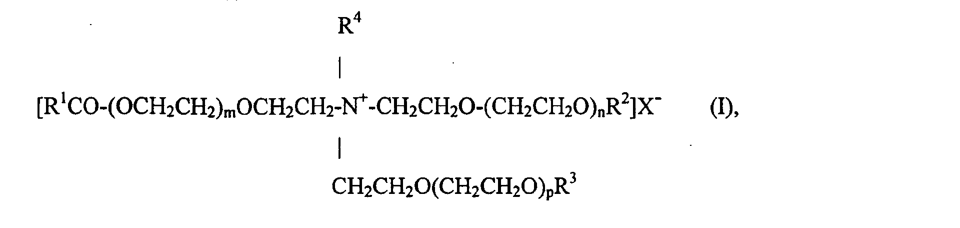 in der R¹CO für einen Acylrest mit 6 bis 22 Kohlenstoffatomen, R² und R³ unabhängig voneinander für Wasserstoff oder R¹CO, R⁴ für einen Alkylrest mit 1 bis 4 Kohlenstoffatomen oder eine (CH₂CH₂O)_{q}H-Gruppe, m, n und p in Summe für 0 oder Zahlen von 1 bis 12, q für Zahlen von 1 bis 12 und X für Halogenid, Alkylsulfat oder Alkylphosphat steht.

Typische Beispiele für diese Esterquats, die im Sinne der Erfindung Verwendung finden können, sind Produkte auf Basis von Capronsäure, Caprylsäure, Caprinsäure, Laurinsäure, Myristinsäure, Palmitinsäure, Isostearinsäure, Stearinsäure, Ölsäure, Elaidinsäure, Arachinsäure, Behensäure und Erucasäure sowie deren technische Mischungen, wie sie beispielsweise bei der Druckspaltung natürlicher Fette und Öle anfallen.

Vorzugsweise werden technische C_{12/14}- und C_{12/18}-Kokosfettsäuren und insbesondere teilgehärtete C_{16/18}-Talg- bzw. Palmfettsäuren sowie elaidinsäurereiche C_{16/18}-Fettsäureschnitte eingesetzt.

Zur Herstellung der quaternierten Ester können die Fettsäuren und das Triethanolamin im molaren Verhältnis von 1,1 : 1 bis 3 : 1 eingesetzt werden. Im Hinblick auf die anwendungstechnischen Eigenschaften der Esterquats hat sich ein Einsatzverhältnis von 1,2 : 1 bis 2,2 : 1, vorzugsweise 1,5 : 1 bis 1,9 : 1 als besonders vorteilhaft erwiesen. Die bevorzugten Esterquats stellen technische Mischungen von Mono-, Di- und Triestern mit einem durchschnittlichen Veresterungsgrad von 1,5 bis 1,9 dar und leiten sich von technischer C_{16/18}- Talg- bzw. Palmfettsäure (Iodzahl 0 bis 40) ab.

Aus anwendungstechnischer Sicht haben sich quaternierte Fettsäuretriethanolaminestersalze der Formel (I) als besonders vorteilhaft erwiesen, in der R¹CO für einen Acylrest mit 16 bis 18 Kohlenstoffatomen, R² für R¹CO, R³ für Wasserstoff, R⁴ für eine Methylgruppe, m, n und p für 0 und X für Methylsulfat steht.

Esterquats, die quaternierte Estersalze von Fettsäuren mit Diethanolalkylaminen darstellen, haben die Formel (II), 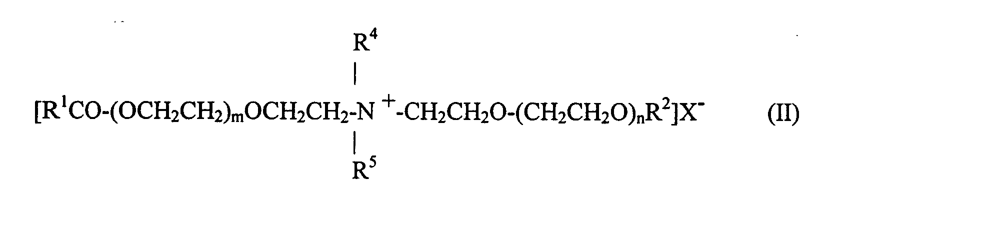 in der R¹CO für einen Acylrest mit 6 bis 22 Kohlenstoffatomen, R² für Wasserstoff oder R¹CO, R⁴ und R⁵ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

Als dritte Gruppe geeigneter Esterquats sind schließlich die quaternierten Estersalze von Fettsäuren mit 1,2-Dihydroxypropyldialkylaminen der Formel (III) zu nennen, 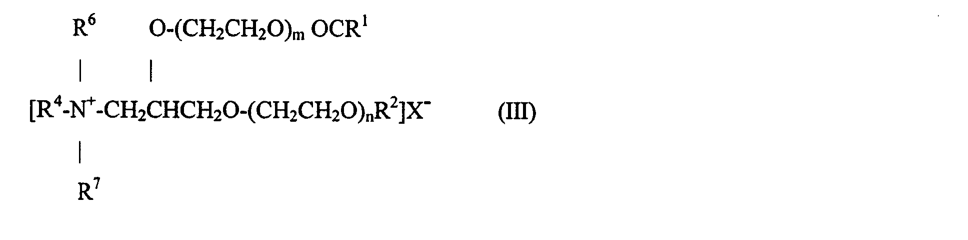 in der R¹CO für einen Acylrest mit 6 bis 22 Kohlenstoffatomen, R² für Wasserstoff oder R¹CO, R⁴, R⁶ und R⁷ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.
Hinsichtlich der Auswahl der bevorzugten Fettsäuren und des optimalen Veresterungsgrades gelten die für (I) genannten Beispiele auch für die Esterquats der Formeln (II) und (III). Esterquats, die in Form 50 bis 90 Gew.- %iger alkoholischer Lösungen im Handel erhältlich sind und problemlos mit Wasser verdünnt werden können, stellen eine bevorzugte eingesetzte Konfektionierungsform dar. In diesem Fall kann Propylenglykol ein bevorzugtes Lösungsmittel für die Esterquats sein.

Die anhand der Formeln I bis III beschriebenen Substanzen stellen naturgemäß Einzelstoffe dar, wobei für den erfindungsgemäßem Einsatz in der Regel auch Gemische dieser Stoffe in Frage kommen können. Ebenso möglich ist gegebenenfalls der Einsatz der technischen Stoffgemische, wie sie aufgrund der Herstellungsverfahren in wechselnder Zusammensetzung erhältlich sind.

Im Rahmen der erfindungsgemäßen Lehre können Esterquats gemäß Formel (II) bevorzugt sein. Eine besonders bevorzugte Verbindung ist das unter der Bezeichnung Armocare® VGH-70 im Handel erhältliche N,N-Bis(2-Palmitoyloxyethyl)dimethylammoniumchlorid. Unter dem Warenzeichen Dehyquart® im Handel erhältliche Esterquats sind insbesondere die Produkte Dehyquart® F-75, Dehyquart® C-4046, Dehyquart® L80 und Dehyquart® AU-35.

Erfindungsgemäß besonders geeignete pseudokationische Tenside sind die Alkylamidoamine der allgemeinen Formel (IV). 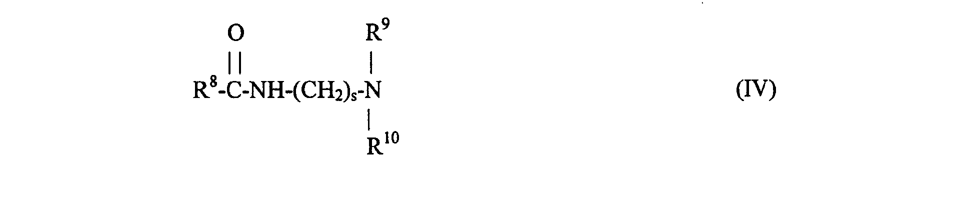 Hierbei steht R⁸-CO für einen aliphatischen, linearen oder verzweigten Acylrest mit 6 bis 22 Kohlenstoffatomen und 0, 1, 2 oder 3 Doppelbindungen, der in der Regel aus einer natürlich vorkommenden oder synthetisch hergestellten Fettsäure stammt. R⁹ und R¹⁰ stellen gleiche oder unterschiedliche Alkylreste dar, die gegebenenfalls funktionelle Gruppen wie Hydroxygruppen, Estergruppen, Amin-, Amid- oder sonstige polare Substituenten tragen können, wobei jedoch in der Regel unsubstituierte Kohlenwasserstoffreste, die allenfalls eine oder mehrere Verzweigungen aufweisen, bevorzugt sind. Insbesondere bevorzugt sind jedoch kurzkettige Kohlenwasserstoffreste mit 1 bis 6 C-Atomen, wobei Ethyl- und Methylreste erfindungsgemäß ganz besonders bevorzugt sind. Die Zahl s ist eine ganze Zahl von 1 bis 10, wobei Werte für n von 2 bis 7, insbesondere von 2 bis 4, bevorzugt sind.

Die Alkylaminoamine werden üblicherweise durch Amidierung natürlicher oder synthetischer Fettsäuren und Fettsäureschnitte mit Dialkylaminoaminen hergestellt. Typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Reduktion von Aldehyden aus der Roelen'schen Oxosynthese oder der Dimerisierung von ungesättigten Fettsäuren anfallen.

Besonders bevorzugt sind die Fettsäureschnitte, welche aus Cocosöl oder Palmöl erhältlich sind; insbesondere bevorzugt ist in der Regel der Einsatz von Stearinsäure.

Eine erfmdungsgemäß besonders geeignete Verbindung aus dieser Substanzgruppe stellt das unter der Bezeichnung Tegoamid® S 18 im Handel erhältliche Stearamidopropyldimethylamin dar.

Die genannten kationischen und pseudokationischen Tenside sind in den erfindungsgemäßen Mitteln bevorzugt in Mengen von 0,2-10 Gew.-%, insbesondere in Mengen von 1-3 Gew.-%, jeweils bezogen auf das gesamte Mittel, enthalten.

Gemäß einer vierten bevorzugten Ausführungsform enthalten die erfindungsgemäßen Mittel weiterhin mindestens ein Ampho-Polymer oder ein kationisches Polymer, ausgewählt aus der Gruppe, die kationische Cellulose-Derivate, kationische Guarderivate, kationische Polymere und Copolymere des Dimethyldiallylammoniumchlorids sowie kationische Copolymere des Vinylimidazoliummethochlorids umfaßt.

Unter dem Oberbegriff Ampho-Polymere sind amphotere Polymere, d.h. Polymere, die im Molekül sowohl freie Aminogruppen als auch freie -COOH- oder SO₃H-Gruppen enthalten und zur Ausbildung innerer Salze befähigt sind, zwitterionische Polymere, die im Molekül quartäre Ammoniumgruppen und -COO⁻- oder -SO₃⁻-Gruppen enthalten, und solche Polymere zusammengefaßt, die -COOH- oder SO₃H-Gruppen und quartäre Ammoniumgruppen enthalten. Ein Beispiel für ein erfindungsgemäß einsetzbares Amphopolymer ist das unter der Bezeichnung Amphomer® erhältliche Acrylharz, das ein Copolymer aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt. Ebenfalls bevorzugte Amphopolymere setzen sich aus ungesättigten Carbonsäuren (z.B. Acryl- und Methacryl-säure), kationisch derivatisierten ungesättigten Carbonsäuren (z.B. Acrylamidopropyl-trimethyl-ammoniumchlorid) und gegebenenfalls weiteren ionischen oder nichtionogenen Monomeren zusammen, wie sie beispielsweise der deutschen Offenlegungsschrift 39 29 973 und dem darin zitierten Stand der Technik zu entnehmen sind. Terpolymere von Acrylsäure, Methylacrylat und Methacrylamidopropyltrimoniumchlorid, wie sie unter der Bezeichnung Merquat®2001 N im Handel erhältlich sind, sind erfindungsgemäß besonders bevorzugte Ampho-Polymere.

Erfindungsgemäß verwendbare kationische Polymere werden aus folgenden Verbindungsklassen ausgewählt:
- quaternisierten Cellulose-Derivaten, wie sie unter den Bezeichnungen Celquat® und Polymer JR® im Handel erhältlich sind. Die Verbindungen Celquat® H 100, Celquat® L 200 und Polymer JR®400 sind bevorzugte quaternierte Cellulose-Derivate.
- kationischen Guar-Derivaten, wie insbesondere die unter den Handelsnamen Cosmedia®Guar und Jaguar® vertriebenen Produkte,
- polymeren Dimethyldiallylammoniumsalzen und deren Copolymeren mit Estern und Amiden von Acrylsäure und Methacrylsäure. Die unter den Bezeichnungen Merquat®100 (Poly(dimethyldiallylammoniumchlorid)) und Merquat®550 (Dimethyldiallylammoniumchlorid-Acrylamid-Copolymer) im Handel erhältlichen Produkte sind Beispiele für solche kationischen Polymere.
- Vinylimidazoliummethochlorid-Copolymeren, insbesondere mit Vinylpyrrolidon, wie sie unter der Bezeichnung Luviquat® angeboten werden.

Die genannten Ampho-Polymer und kationischen Polymere sind in den erfindungsgemäßen Mitteln bevorzugt in Mengen von 0,01-5 Gew.-%, insbesondere in Mengen von 0,05-2 Gew.-%, jeweils bezogen auf das gesamte Mittel, enthalten.

Besonders hervorragende Eigenschaften entfalten die erfindungsgemäß verwendeten Wirkstoffe auch in Kombination mit bestimmten anionischen Tensiden. Gemäß einer vierten bevorzugten Ausführungsform enthalten die erfindungsgemäßen Mittel weiterhin mindestens ein anionisches Tensid, ausgewählt aus C₆-C₂₀-Acylglutamaten, Ethercarbonsäuresalzen sowie Estern von Hydroxydi- und -tricarbonsäuren mit polyhydroxylierten Verbindungen.

Ein erfindungsgemäß bevorzugtes Acylglutamat ist das Handelsprodukt Protelan® AGL 95 (Tschimmer & Schwarz).

Erfindungsgemäß verwendbare Ethercarbonsäuresalze sind insbesondere, jeweils in Form ihrer Natrium-, Kalium-, Magnesium- und Ammonium- sowie der Mono-, Di- und Trialkanolammoniumsalze mit 2 oder 3 C-Atomen in der Alkanolgruppe, Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 16 ist. Solche Verbindungen sind beispielsweise unter dem Warenzeichen Akypo® im Handel erhältlich.

Erfindungsgemäß verwendbare Ester von Hydroxydi- und -tricarbonsäuren mit polyhydroxylierten Verbindungen sind insbesondere solche mit der allgemeinen Formel (V), 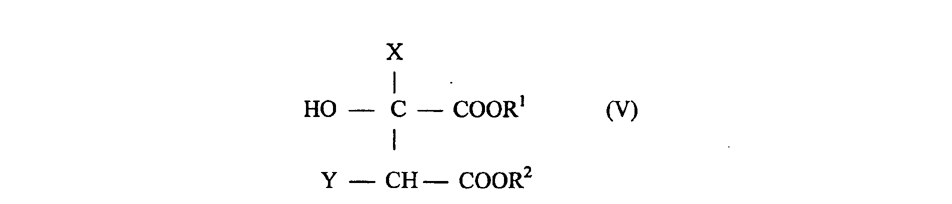 in der
X H oder eine -CH₂COOR-Gruppe ist,
Y H oder -OH ist unter der Bedingung, daß Y H ist, wenn X -CH₂COOR ist,
R, R¹ und R² unabhängig voneinander ein Wasserstoffatom, ein Alkali- oder Erdalkalimetallkation, eine Ammoniumgruppe, das Kation einer ammonium-organischen Base oder einen Rest Z, der von einer polyhydroxylierten organischen Verbindung stammt, die aus der Gruppe, die veretherte (C₆-C₁₈)-Alkyl-Polysaccharide mit 1 bis 6 monomeren Saccharideinheiten und veretherte aliphatische (C₆-C₁₆)-Hydroxyalkyl-Polyole mit 2 bis 16 Hydroxylresten umfaßt, ausgewählt ist, bedeuten, unter der Bedingung, daß mindestens eine der Gruppen R, R¹ oder R² ein Rest Z ist.

Die Verbindungen der Formel (V) sind beispielsweise aus der Europäischen Patentschrift EP-B1-0 258 814 sowie aus den Artikeln von N. Burns, Drug Cosmet. Ind. 160(3), 42 (1997) und T. Verzotti et al., Cosmetic News XX-112, 29 (1997) bekannt.Bezüglich der einzelnen Vertreter, die durch die allgemeine Formel (V) definiert werden, wird ausdrücklich auf den Inhalt dieser Europäischen Patentschrift, insbesondere die Passage von Seite 2, Zeile 43, bis Seite 5, Zeile 56, Bezug genommen.

Im Rahmen der vorliegenden Erfindung stehen R, R¹ und R² bevorzugt für Alkalimetallkationen, insbesondere das Natriumion, Erdalkalimetallkationen, insbesondere das Magnesiumion, und das Ammoniumion.

Die Gruppe Z ist bevorzugt ein verethertes (C₆-C₁₈)-Alkyl-Polysaccharid.
Als Saccharid enthält die Gruppe Z bevorzugt Glucose.
Als Alkylgruppen werden insbesondere C₆-C₁₆-Alkylgruppen eingesetzt, wobei die Wahl von unverzweigten gesättigten Gruppen bevorzugt ist. Bei den Alkylgruppen kann es sich aber auch um Mischungen handeln, die bei der Verarbeitung natürlicher Fette und Öle erhalten werden. Mischungen, bestehend im wesentlichen aus
- C₈-C₁₀-Alkylgruppen,
- C₁₂-C₁₄-Alkylgruppen oder
- C₈-C₁₆-Alkylgruppen
können erfindungsgemäß bevorzugt sein. Ganz besonders bevorzugt ist eine Mischung von Alkylgruppen, wie sie bei der Verarbeitung von Kokosöl erhalten wird.
Die Gruppe Z enthält 1 bis 6 monomere Saccharideinheiten. Gemäß einer bevorzugten Ausführungsform enthält die Gruppe Z 2 bis 6 Glucoseeinheiten. Es kann aber auch bevorzugt sein, Gruppen Z mit 1,2 bis 3, insbesondere 1,3 bis 2, Glucoseeinheiten einzusetzen. Dabei ist zu berücksichtigen, daß bei der Synthese immer Mischungen entstehen, und diese Zahlenwerte aus dem stöchiometrischen Verhältnis der Ausgangssubstanzen Fettalkohol und Saccharid hergeleitet werden.

Die Verbindungen gemäß Formel (V) sind Derivate der Zitronensäure, der Weinsäure oder der Äpfelsäure. Solche Verbindungen, die Derivate der Zitronensäure und insbesondere der Weinsäure darstellen, sind erfindungsgemäß bevorzugt.

Handelsprodukte, die von der Firma Cesalpinia unter den Bezeichnungen Eucarol® AGE-ET und Eucarol® AGE-EC vertrieben werden, haben sich als besonders gut geeignete Vertreter der Verbindungen gemäß Formel (V) erwiesen.

Die genannten anionischen Tenside sind in den erfindungsgemäßen Mitteln bevorzugt in Mengen von 0,5-10 Gew.-%, insbesondere in Mengen von 1-5 Gew.-%, jeweils bezogen auf das gesamte Mittel, enthalten.

Ebenfalls hervorragende Eigenschaften entfalten die erfindungsgemäß verwendeten Wirkstoffe in Kombination mit Silikonen. Gemäß einer fünften bevorzugten Ausführungsform enthalten die erfindungsgemäßen Mittel weiterhin mindestens ein Silikon.

Erfindungsgemäß geeignete Silikone oder Silikongums sind insbesondere Dialkyl- und Alkylarylsiloxane, wie beispielsweise Dimethylpolysiloxan und Methylphenyl-polysiloxan, sowie deren alkoxylierte, quaternierte oder auch anionische Derivate.

Beispiele für solche Silikone sind:
- oligomere Polydimethylcyclosiloxane (INCI-Bezeichnung: Cyclomethicone), insbesondere die pentamere Verbindung, die als Handelsprodukt DC 345 von Dow Corning vertrieben wird,
- Hexamethyl-Disiloxan (INCI-Bezeichnung: Hexamethyldisiloxane), z. B. das unter der Bezeichnung Abil® K 520 vertriebenen Produkt,
- polymere Polydimethylsiloxane (INCI-Bezeichnung: Dimethicone), z. B. die unter der Bezeichnung DC 200 von Dow Corning vertriebenen Produkte,
- Alkylmethylpolysiloxane, z. B. das unter der Bezeichnung Silsoft® 034 vertriebene Produkt mit der INCI-Bezeichnung Caprylyl Methicone,
- Polyphenylmethylsiloxane (INCI-Bezeichnung: Phenyl Trimethicone), z. B. das Handelsprodukt DC 556 Fluid von Dow Corning,
- Silicon-Glykol-Copolymere (INCI-Bezeichnung: Dimethicone Copolyol), z. B. die Handelsprodukte DC 190 und DC 193 von Dow Corning, sowie deren Derivate, z. B das Handelsprodukt Silsoft® Shine (INCI-Bezeichnung: Methyleugenyl Dimethicone Copolyol),
- Veresterte Silicon-Glykol-Copolymere, wie beispielsweise die Handelsprodukte Fancorsil® LIM-1, LIM-2 und LIM-3 mit der INCI-Bezeichnung Dimethicone Copolyol Meadowfoamate,
- Dimethylsiloxane mit Hydroxy-Endgruppen (INCI-Bezeichnung: Dimethiconol), z. B. die Handelsprodukte DC 1401 und Q2-1403 von Dow Corning,
- aminofunktionelle Polydimethylsiloxane und hydroxylaminomodifizierte Silicone (INCI-Bezeichnung: u. a. Amodimethicone und Quatemium-80), wie die Handelsprodukte XF42-B1989 (Hersteller GE Toshiba Silicones) Q2-7224 (Hersteller: Dow Corning; ein stabilisiertes Trimethylsilylamodimethicon), Dow Corning® 939 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt) sowie das Handelsprodukt Silsoft® A-843,
- anionische Silikonöle, wie beispielsweise das Produkt Dow Corning®1784.

Gemäß einer bevorzugten Ausführungsform können die erfindungsgemäßen Zubereitungen eine Kombination aus einem flüchtigen und einem nichtflüchtigen Silikon enthalten. Flüchtig im Sinne der Erfindung sind solche Silikone, die ein Flüchtigkeit aufweisen, die gleich oder größer als die Flüchtigkeit des cyclischen, pentameren Dimethylsiloxans ist. Solche Kombinationen sind auch als Handelsprodukte (z. B. Dow Corning®1401, Dow Corning®1403 und Dow Corning®1501, jeweils Mischungen aus einem Cyclomethicon und einem Dimethiconol) erhältlich.

Die erfindungsgemäßen Zubereitungen enthalten die Silikone bevorzugt in Mengen von 0,05 - 10 Gew.-%, insbesondere 0,1 - 5 Gew.-%, bezogen auf die gesamte Anwendungszubereitung.

Gemäß einer, ebenfalls bevorzugten, sechsten Ausführungsform enthalten die erfindungsgemäßen Mittel weiterhin einen Ölkörper, ausgewählt aus flüssigen Paraffinölen, Isoparaffinölen, synthetischen Kohlenwasserstoffen, Di-n-alkylethern, Esterölen, Kohlensäureestern mit Fettalkoholen, alkoxylierten Monoglyceriden sowie Dimeren, Telomeren und Polymeren von Olefinen.

Zu den Ölkörpern, welche die Wirkung des erfindungsgemäßen Wirkstoffes steigern können, sind zu zählen:
- flüssige Paraffinöle, Isoparaffinöle und synthetische Kohlenwasserstoffe sowie Di-nalkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol® S) und Di-n-octylether (Cetiol® OE) können bevorzugt sein.
- Esteröle. Unter Esterölen sind zu verstehen die Ester von C₆ - C₃₀ - Fettsäuren mit C₂ - C₃₀ - Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat (Rilanit® IPM), Isononansäure-C16-18-alkylester (Cetiol® SN), 2-Ethylhexylpalmitat (Cegesoft® 24), Stearinsäure-2-ethylhexylester (Cetiol® 868), Cetyloleat, Glycerintricaprylat, Kokosfettalkohol-caprinat/-caprylat (Cetiol® LC), n-Butylstearat, Oleylerucat (Cetiol® J 600), Isopropylpalmitat (Rilanit® IPP), Oleyloleat (Cetiol® DAB), Laurinsäurehexylester (Cetiol® A), Di-n-butyladipat (Cetiol® B), Myristylmyristat (Cetiol® MM), Cetearyl Isononanoate (Cetiol® SN), Ölsäuredecylester (Cetiol® V).
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol® CC),
- alkoxylierte Monoglyceride, wie beispielsweise das Handelsprodukt Cetiol® HE.
- Dimere, Telomere und Polymere von Olefinen sind insbesondere aus niedermolekularen Olefinen wie Ethylen, Propylen, Butylen, Isobutylen, Penten, Cyclopenten, Isopentenen oder 1-Decen aufgebaute Dimere, Telomere und Polymere, welche gegebenenfalls noch hydriert sein können. Derartige Produkte können beispielsweise unter den Handelsbezeichnungen Nexbase®, Permethyl®, beispielsweise Permethyl® 102A, Gulftene®, zum Beispiel Gulftene® 10 oder Gulftene® 12, Arlamol® PA01, Paradecinol®, wie beispielsweise insbesondere Paradecinol® FV 16-18, oder Indopol® käuflich erworben werden.

Die Einsatzmenge beträgt 0,1 - 50 Gew.% bezogen auf das gesamte Mittel, bevorzugt 0,1 - 20 Gew.% und besonders bevorzugt 0,1 - 15 Gew.% bezogen auf das gesamte Mittel.

Auch durch die Kombination mit Proteinhydrolysaten und deren Derivaten kann die Wirkung der erfindungsgemäß verwendeten Wirkstoffe (W) in überraschender Weise gesteigert werden. Gemäß einer siebten bevorzugten Ausführungsform enthalten die erfindungsgemäßen Mittel weiterhin mindestens ein Proteinhydrolysat oder dessen Derivat.

Proteinhydrolysate sind Produktgemische, die durch sauer, basisch oder enzymatisch katalysierten Abbau von Proteinen (Eiweißen) erhalten werden.

Erfindungsgemäß können Proteinhydrolysate sowohl pflanzlichen als auch tierischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan® (Cognis), Promois® (Interorgana), Collapuron® (Cognis), Nutrilan® (Cognis), Gelita-Sol® (Deutsche Gelatine Fabriken Stoess & Co), Lexein® (Inolex) und Kerasol® (Croda) vertrieben.

Erfindungsgemäß bevorzugt ist die Verwendung von Proteinhydrolysaten pflanzlichen Ursprungs, z. B. Soja-, Mandel-, Reis-, Erbsen-, Kartoffel- und Weizenproteinhydrolysate. Solche Produkte sind beispielsweise unter den Warenzeichen Gluadin® (Cognis), DiaMin® (Diamalt), Lexein® (Inolex) und Crotein® (Croda) erhältlich.

Wenngleich der Einsatz der Proteinhydrolysate als solche bevorzugt ist, können an deren Stelle gegebenenfalls auch anderweitig erhaltene Aminosäuregemische oder einzelne Aminosäuren wie beispielsweise Arginin, Lysin, Histidin oder Pyroglutaminsäure eingesetzt werden. Ebenfalls möglich ist der Einsatz von Derivaten der Proteinhydrolysate, beispielsweise in Form ihrer Fettsäure-Kondensationsprodukte. Solche Produkte werden beispielsweise unter den Bezeichnungen Lamepon® (Cognis), Gluadin® (Cognis), Lexein® (Inolex), Crolastin® (Croda) oder Crotein® (Croda) vertrieben.

Erfindungsgemäß einsetzbar sind auch kationisierte Proteinhydrolysate, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder von biotechnologisch gewonnenen Proteinhydrolysaten, stammen kann. Die den erfindungsgemäßen kationischen Derivaten zugrunde liegenden Proteinhydrolysate können aus den entsprechenden Proteinen durch eine chemische, insbesondere alkalische oder saure Hydrolyse, durch eine enzymatische Hydrolyse und/oder einer Kombination aus beiden Hydrolysearten gewonnen werden. Die Hydrolyse von Proteinen ergibt in der Regel ein Proteinhydrolysat mit einer Molekulargewichtsverteilung von etwa 100 Dalton bis hin zu mehreren tausend Dalton. Bevorzugt sind solche kationischen Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Die Quaternisierung der Proteinhydrolysate oder der Aminosäuren wird häufig mittels quarternären Ammoniumsalzen wie beispielsweise N,N-Dimethyl-N-(n-Alkyl)-N-(2-hydroxy-3-chloro-n-propyl)-ammoniumhalogeniden durchgeführt. Weiterhin können die kationischen Proteinhydrolysate auch noch weiter derivatisiert sein. Als typische Beispiele für die erfindungsgemäßen kationischen Proteinhydrolysate und -derivate seien die unter den INCI - Bezeichnungen im "International Cosmetic Ingredient Dictionary and Handbook", (seventh edition 1997, The Cosmetic, Toiletry, and Fragrance Association 1101 17^{th} Street, N.W., Suite 300, Washington, DC 20036-4702) genannten und im Handel erhältlichen Produkte genannt: Cocodimonium Hydroxypropyl Hydrolyzed Collagen, Cocodimonium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Silk, Cocodimonium Hydroxypropyl Hydrolyzed Soy Protein, Cocodimonium Hydroxypropyl Hydrolyzed Wheat Protein, Cocodimonium Hydroxypropyl Silk Amino Acids, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed Keratin, Hydroxypropyltrimonium Hydrolyzed Rice Bran Protein, Hydroxyproypltrimonium Hydrolyzed Silk, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Silk, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Silk, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Silk, Quaternium-79 Hydrolyzed Soy Protein, Quaternium-79 Hydrolyzed Wheat Protein. Ganz besonders bevorzugt sind die kationischen Proteinhydrolysate und -derivate auf pflanzlicher Basis.

In den erfindungsgemäß verwendeten Mitteln sind die Proteinhydrolysate und deren Derivate in Mengen von 0,01 - 10 Gew.-% bezogen auf das gesamte Mittel enthalten. Mengen von 0,1 bis 5 Gew.%, insbesondere 0,1 bis 3 Gew.-%, sind ganz besonders bevorzugt.

Gemäß einer achten Ausführungsform enthalten die erfindungsgemäßen Mittel weiterhin mindestens einen Feststoff, insbesondere mindestens einen Fettstoff, in nanopartikulärer Form. Ein solcher Feststoff ist beispielsweise hydriertes Rizinusöl. Die Größe der Nanopartikel liegt dabei bevorzugt bei etwa 100 nm oder darunter.

Hinsichtlich der Art, gemäß der der erfindungsgemäße verwendete Wirkstoff beziehungsweise die erfindungsgemäßen Wirkstoffkombinationen auf das Haar und/oder auf die Haut aufgebracht werden, bestehen keine prinzipiellen Einschränkungen. Als Konfektionierung dieser Zubereitungen sind beispielsweise Cremes, Lotionen, Lösungen, Wässer, Emulsionen wie W/O-, O/W-, PIT-Emulsionen (Emulsionen nach der Lehre der Phaseninversion, PIT genannt), Mikroemulsionen und multiple Emulsionen, grobe, instabile, ein oder mehrphasige Schüttelmixturen, Gele, Sprays, Aerosole und Schaumaerosole geeignet. Diese werden in der Regel auf wäßriger oder wäßrigalkoholischer Basis formuliert. Als alkoholische Komponente kommen dabei niedere Alkanole sowie Polyole wie Propylenglykol und Glycerin zum Einsatz. Ethanol und Isopropanol sind bevorzugte Alkohole. Wasser und Alkohol können in der wäßrig alkoholischen Basis in einem Gewichtsverhältnis von 1 : 10 bis 10 : 1 vorliegen. Wasser sowie wäßrig-alkoholische Mischungen, die bis zu 50 Gew.-%, insbesondere bis zu 25 Gew.-%, Alkohol, bezogen auf das Gemisch Alkohol/Wasser, enthalten, können erfindungsgemäß bevorzugte Grundlagen sein. Der pH-Wert dieser Zubereitungen kann prinzipiell bei Werten von 2 - 11 liegen. Er liegt bevorzugt zwischen 2 und 7, wobei Werte von 4 bis 6 besonders bevorzugt sind. Zur Einstellung dieses pH-Wertes kann praktisch jede für kosmetische Zwecke verwendbare Säure oder Base verwendet werden. Üblicherweise werden als Säuren Genußsäuren verwendet. Unter Genußsäuren werden solche Säuren verstanden, die im Rahmen der üblichen Nahrungsaufnahme aufgenommen werden und positive Auswirkungen auf den menschlichen Organismus haben. Genußsäuren sind beispielsweise Essigsäure, Milchsäure, Weinsäure, Zitronensäure, Äpfelsäure, Ascorbinsäure und Gluconsäure. Im Rahmen der Erfindung ist die Verwendung von Zitronensäure und Milchsäure besonders bevorzugt. Bevorzugte Basen sind Ammoniak, Alkalihydroxide, Monoethanolamin, Triethanolamin sowie N,N,N',N'-Tetrakis-(2-hydroxypropyl)-ethylendiamin.

In einer besonderen Ausführungsform der erfindungsgemäßen Mittel kann es bevorzugt sein, wenn die Mittel als Mikroemulsion vorliegen. Unter Mikroemulsionen werden im Rahmen der Erfindung ebenfalls sogenannte "PIT"-Emulsionen verstanden. Bei diesen Emulsionen handelt es sich im Prinzip um Systeme mit den 3 Komponenten Wasser, Öl und Emulgator, die bei Raumtemperatur als Öl-in-Wasser(O/W)-Emulsion vorliegen. Beim Erwärmen dieser Systeme bilden sich in einem bestimmten Temperaturbereich (üblicherweise als Phaseninversiontemperatur oder "PIT" bezeichnet) Mikroemulsionen aus, die sich bei weiterer Erwärmung in Wasser-in-Öl(W/O)-Emulsionen umwandeln. Bei anschließendem Abkühlen werden wieder O/W-Emulsionen gebildet, die aber auch bei Raumtemperatur als Mikroemulsionen mit einem mittleren Teilchendurchmesser von kleiner als 400 nm, insbesondere mit einem Teilchendurchmesser von etwa 100-300 nm, vorliegen. Einzelheiten bezüglich dieser sehr stabilen, niedrigviskosen Systeme, für die sich die Bezeichnung "PIT-Emulsionen" allgemein durchgesetzt hat, sind einer Vielzahl von Druckschriften zu entnehmen, für die stellvertretend die Veröffentlichungen in Angew. Chem. 97, 655-669 (1985) und Adv. Colloid Interface Sci 58, 119-149 (1995) genannt werden.

Erfindungsgemäß können solche Mikro- oder "PIT"-Emulsionen bevorzugt sein, die einen mittleren Teilchendurchmesser von etwa 200 nm aufweisen.

Die Herstellung der erfindungsgemäßen Mikroemulsionen kann beispielsweise in der Art erfolgen, daß zunächst die Phaseninversionstemperatur des Systems bestimmt wird, indem man eine Probe der auf übliche Weise hergestellten Emulsion erhitzt und unter Verwendung eines Leitfähigkeitsmeßgerätes die Temperatur bestimmt, bei der die Leitfahigkeit stark abnimmt. Die Abnahme der spezifischen Leitfähigkeit der zunächst vorhandenen O/W-Emulsion nimmt dabei in der Regel über einen Temperaturbereich von 2 bis 8 °C von ursprünglich mehr als 1 mS/cm auf Werte unterhalb von 0,1 mS/cm ab. Dieser Temperaturbereich entspricht dann dem Phaseninversions-Temperaturbereich. Nachdem somit der Phaseninversions-Temperaturbereich bekannt ist, kann man die zunächst wie üblich hergestellte Emulsion aus Ölkomponente, nichtionogenem Emulgator, zumindest Teilen des Wassers sowie gegebenenfalls weiteren Komponenten auf eine Temperatur erhitzen, die innerhalb oder oberhalb des Phaseninversions-Temperaturbereiches liegt, sodann abkühlen und gegebenenfalls weitere Komponenten sowie das restliche Wasser hinzufügen. Alternativ kann auch die Herstellung der Mikroemulsion direkt bei einer Temperatur erfolgen, die innerhalb oder oberhalb des Phaseninversions-Temperaturbereiches liegt. Die so hergestellte Mikroemulsion wird dann auf eine Temperatur unterhalb des Phaseninversions-Temperaturbereiches, üblicherweise Raumtemperatur, abgekühlt.

Die erfindungsgemäßen Mittel können nach einer Einwirkzeit wieder von dem Haar bzw. der Haut entfernt werden oder sie können auf dem Haar oder der Haut verbleiben. Unter auf der Haut und dem Haar verbleibend werden erfindungsgemäß solche Zubereitungen verstanden, die nicht im Rahmen der Behandlung nach einem Zeitraum von wenigen Sekunden bis zu einer Stunde mit Hilfe von Wasser oder einer wäßrigen Lösung wieder von der Haut ab- oder aus dem Haar ausgespült werden. Vielmehr verbleiben die Zubereitungen bis zur nächsten Wäsche auf der Haut oder dem Haar.

Bei den erfindungsgemäßen Mitteln kann es sich beispielsweise um reinigende Mittel für Haut und Haar wie Shampoos, Duschbäder, Schaumbäder, Flüssigseifen, Make-upremover und Gesichtsreiniger, um pflegende Mittel für Haut und Haar wie Spülungen, Haarwässer, Tagescremes, Nachtcremes und Gesichtsmasken, um festigende Mittel für das Haar wie Haarfestiger, Schaumfestiger, Styling Gels und Fönwellen, dauerhafte Verformungsmittel wie Dauerwell- und Fixiermittel, um im Rahmen eines Dauerwellverfahrens oder Färbeverfahrens eingesetzte Vorbehandlungsmittel oder Nachspülungen, sowie um permanente und temporäre Färbemittel insbesondere für Haare handeln.

Entsprechend der Art des Mittels enthalten diese dann jeweils die für diese Mittel üblichen weiteren Komponenten.

Insbesondere reinigende Mittel wie Shampoos, Duschbäder, Schaumbäder und Flüssigseifen enthalten in der Regel noch mindestens eine weitere reinigende Komponente. Diese wird insbesondere aus der Gruppe der anionischen, ampholytischen, zwitterionischen und nichtionogenen Tenside ausgewählt; es kann sich aber auch um kationische Tenside handeln.

Als anionische Tenside eignen sich in erfindungsgemäßen Mitteln alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium-, Magnesium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Amidethercarboxylate der Formel [R-NH(-CH₂-CH₂-O)ₙ-CH₂-COO]ₘZ, in der R für einen linearen oder verzweigten, gesättigten oder ungesättigten Acylrest mit 2 bis 29 C-Atomen, n für ganze Zahlen von 1 bis 10, m für die Zahlen 1 oder 2 und Z für ein Kation aus der Gruppe der Alkali- oder Erdalkalimetalle steht,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und dialkylester mit 8 bis 18 C-Atomen in der Alkylgruppe und Sulfobemsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(-CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Kokosmonoglyceridsulfate.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül, sowie Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel RO-(S)ₓ. Diese Verbindungen sind durch die folgenden Parameter gekennzeichnet.

Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegend Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest R enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen besteht.

Als Zuckerbaustein S können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Die Alkylglykoside können neben ihrer Tensidwirkung auch dazu dienen, die Fixierung der Duftkomponenten auf dem Haar zu verbessern. Der Fachmann wird also für den Fall, daß eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weiterhin können, insbesondere als Co-Tenside, zwitterionische Tenside verwendet werden. Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Weitere erfindungsgemäß verwendbare kationische Tenside stellen die quaternisierten Proteinhydrolysate dar.

Vorzugsweise enthalten die erfindungsgemäßen Shampoos die weiteren reinigenden Komponenten in Mengen von 5,0 bis 40 Gew.-%, insbesondere von 5,0 bis 20 Gew.-%, bezogen auf die jeweilige Zubereitung.

Die erfindungsgemäßen Mittel können weiterhin noch zusätzliche Polymer-Komponenten enthalten.

Bevorzugte kationische Polymere sind beispielsweise
- Polysiloxane mit quaternären Gruppen, wie beispielsweise die im Handel erhältlichen Produkte Q2-7224 (Hersteller: Dow Corning; ein stabilisiertes Trimethylsilylamodimethicon), Dow Corning® 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydime- thylsiloxane, Quaternium-80),
- Copolymere des Vinylpyrrolidons mit quaternierten Derivaten des Dialkylaminoacrylats und -methacrylats, wie beispielsweise mit Diethylsulfat quaternierte Vinylpyrrolidon-Dimethylaminomethacrylat-Copolymere. Solche Verbindungen sind unter den Bezeichnungen Gafquat®734 und Gafquat®755 im Handel erhältlich.
- quaternierter Polyvinylalkohol
   sowie die unter den Bezeichnungen
- Polyquaternium-2,
- Polyquaternium-17,
- Polyquaternium-18 und
- Polyquaternium-27
bekannten Polymeren mit quartären Stickstoffatomen in der Polymerhauptkette.

In einer weiteren Ausführungsform wird der erfindungsgemäß verwendete Wirkstoff (W) in Mitteln zum Färben keratinischer Fasern eingesetzt. Dabei kann der erfindungsgemäße Wirkstoff prinzipiell dem Färbemittel direkt zugegeben werden oder das Aufbringen des Wirkstoff-haltigen Mittels erfolgt auf die gefärbte keratinische Faser in einem getrennten Schritt entweder direkt im Anschluß an den eigentlichen Färbevorgang oder in getrennten Behandlungen, gegebenenfalls auch Tage oder Wochen nach dem Färbevorgang.

Der Begriff Färbevorgang umfaßt dabei alle dem Fachmann bekannten Verfahren, bei denen auf das, gegebenenfalls angefeuchtete, Haar ein Färbemittel aufgebracht wird und dieses entweder für eine Zeit zwischen wenigen Minuten und ca. 45 Minuten auf dem Haar belassen und anschließend mit Wasser oder einem tensidhaltigen Mittel ausgespült wird oder ganz auf dem Haar belassen wird. Es wird in diesem Zusammenhang ausdrücklich auf die bekannten Monographien, z. B. Kh. Schrader, Grundlagen und Rezepturen der Kosmetika, 2. Auflage, Hüthig Buch Verlag, Heidelberg, 1989, verwiesen, die das entsprechende Wissen des Fachmannes wiedergeben.

Wie bereits zuvor erwähnt ist es im Rahmen der erfindungsgemäßen Lehre möglich, den Wirkstoff direkt in die Färbe- oder Tönungsmittel einzuarbeiten.

Die Zusammensetzung des Färbe- oder Tönungsmittels unterliegt keinen prinzipiellen Einschränkungen.
Als Farbstoff(vorprodukt)e können
- Oxidationsfarbstoffvorprodukte vom Entwickler- und Kuppler-Typ,
- natürliche und synthetische direktziehende Farbstoffe und
- Vorstufen naturanaloger Farbstoffe, wie Indol- und Indolin-Derivate,
sowie Mischungen von Vertretern einer oder mehrerer dieser Gruppen eingesetzt werden.

Als Oxidationsfarbstoffvorprodukte vom Entwickler-Typ werden üblicherweise primäre aromatische Amine mit einer weiteren, in para- oder ortho-Position befindlichen, freien oder substituierten Hydroxy- oder Aminogruppe, Diaminopyridinderivate, heterocyclische Hydrazone, 4-Aminopyrazolderivate sowie 2,4,5,6-Tetraaminopyrimidin und dessen Derivate eingesetzt. Geeignete Entwicklerkomponenten sind beispielsweise p-Phenylendiamin, p-Toluylendiamin, p-Aminophenol, o-Aminophenol, 1-(2'-Hydroxyethyl)-2,5-diaminobenzol, N,N-Bis-(2-hydroxy-ethyl)-p-phenylendiamin, 2-(2,5-Diaminophenoxy)-ethanol, 4-Amino-3-methylphenol, 2,4,5,6-Tetraaminopyrimidin, 2-Hydroxy-4,5,6-triaminopyrimidin, 4-Hydroxy-2,5,6-triaminopyrimidin, 2,4-Dihydroxy-5,6-diaminopyrimidin, 2-Dimethylamino-4,5,6-triaminopyrimidin, 2-Hydroxymethylamino-4-amino-phenol, Bis-(4-aminophenyl)amin, 4-Amino-3-fluorphenol, 2-Aminomethyl-4-aminophenol, 2-Hydroxymethyl-4-aminophenol, 4-Amino-2-((diethylamino)-methyl)-phenol, Bis-(2-hydroxy-5-aminophenyl)-methan, 1,4-Bis-(4-aminophenyl)-diazacycloheptan, 1,3-Bis(N(2-hydroxyethyl)-N(4-aminophenylamino))-2-propanol, 4-Amino-2-(2-hydroxyethoxy)-phenol, 1,10-Bis-(2,5-diaminophenyl)-1,4,7,10-tetraoxadecan sowie 4,5-Diaminopyrazol-Derivate nach EP 0 740 741 bzw. WO 94/08970 wie z. B. 4,5-Diamino-1-(2'-hydroxyethyl)-pyrazol. Besonders vorteilhafte Entwicklerkomponenten sind p-Phenylendiamin, p-Toluylendiamin, p-Aminophenol, 1-(2'-Hydroxyethyl)-2,5-diaminobenzol, 4-Amino-3-methylphenol, 2-Aminomethyl-4-aminophenol, 2,4,5,6-Tetraaminopyrimidin, 2-Hydroxy-4,5,6-triaminopyrimidin, 4-Hydroxy-2,5,6-triaminopyrimidin.

Als Oxidationsfarbstoffvorprodukte vom Kuppler-Typ werden in der Regel m-Phenylendiaminderivate, Naphthole, Resorcin und Resorcinderivate, Pyrazolone und m-Aminophenolderivate verwendet. Beispiele für solche Kupplerkomponenten sind
- m-Aminophenol und dessen Derivate wie beispielsweise 5-Amino-2-methylphenol, 5-(3-Hydroxypropylamino)-2-methylphenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-(Ethylamino)-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2,4-diaminophenoxy)-propan, 1-Methoxy-2-amino-4-(2'-hydroxyethylamino)benzol, 1,3-Bis-(2,4-diaminophenyl)-propan, 2,6-Bis-(2-hydroxyethylamino)-1-methylbenzol und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Amino-benzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol,

Besonders geeignete Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Direktziehende Farbstoffe sind üblicherweise Nitrophenylendiamine, Nitroaminophenole, Azofarbstoffe, Anthrachinone oder Indophenole. Besonders geeignete direktziehende Farbstoffe sind die unter den internationalen Bezeichnungen bzw. Handelsnamen HC Yellow 2, HC Yellow 4, HC Yellow 5, HC Yellow 6, Basic Yellow 57, Disperse Orange 3, HC Red 3, HC Red BN, Basic Red 76, HC Blue 2, HC Blue 12, Disperse Blue 3, Basic Blue 99, HC Violet 1, Disperse Violet 1, Disperse Violet 4, Disperse Black 9, Basic Brown 16 und Basic Brown 17 bekannten Verbindungen sowie 1,4-Bis-(β-hydroxyethyl)-amino-2-nitrobenzol, 4-Amino-2-nitrodiphenylamin-2'-carbonsäure, 6-Nitro-1,2,3,4-tetrahydrochinoxalin, Hydroxyethyl-2-nitro-toluidin, Pikraminsäure, 2-Amino-6-chloro-4-nitrophenol, 4-Ethylamino-3-nitrobenzoesäure und 2-Chloro-6-ethylamino-1-hydroxy-4-nitrobenzol.

In der Natur vorkommende direktziehende Farbstoffe sind beispielsweise die in Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthaltenen färbenden Verbindungen.
Es ist nicht erforderlich, daß die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Als Vorstufen naturanaloger Farbstoffe werden beispielsweise Indole und Indoline sowie deren physiologisch verträgliche Salze verwendet. Bevorzugt werden solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. Besonders vorteilhafte Eigenschaften haben 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure, 6-Hydroxyindolin, 6-Aminoindolin und 4-Aminoindolin sowie 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin sowie N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate in den im Rahmen des erfindungsgemäßen Verfahrens eingesetzten Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden.

Bei der Verwendung von Farbstoff-Vorstufen vom Indolin- oder Indol-Typ kann es bevorzugt sein, diese zusammen mit mindestens einer Aminosäure und/oder mindestens einem Oligopeptid einzusetzen. Bevorzugte Aminosäuren sind Aminocarbonsäuren, insbesondere α-Aminocarbonsäuren und ω-Aminocarbonsäuren. Unter den α-Aminocarbonsäuren sind wiederum Arginin, Lysin, Ornithin und Histidin besonders bevorzugt. Eine ganz besonders bevorzugte Aminosäure ist Arginin, insbesondere in freier Form, aber auch als Hydrochlorid eingesetzt.

Haarfärbemittel, insbesondere wenn die Ausfärbung oxidativ, sei es mit Luftsauerstoff oder anderen Oxidationsmitteln wie Wasserstoffperoxid, erfolgt, werden üblicherweise schwach sauer bis alkalisch, d. h. auf pH-Werte im Bereich von etwa 5 bis 11, eingestellt. Zu diesem Zweck enthalten die Färbemittel Alkalisierungsmittel, üblicherweise Alkali- oder Erdalkalihydroxide, Ammoniak oder organische Amine. Bevorzugte Alkalisierungsmittel sind Monoethanolamin, Monoisopropanolamin, 2-Amino-2-methyl-propanol, 2-Amino-2-methyl-1,3-propandiol, 2-Amino-2-ethyl-1,3-propandiol, 2-Amino-2-methylbutanol und Triethanolamin sowie Alkali- und Erdalkalimetallhydroxide. Insbesondere Monoethanolamin, Triethanolamin sowie 2-Amino-2-methyl-propanol und 2-Amino-2-methyl-1,3-propandiol sind im Rahmen dieser Gruppe bevorzugt. Auch die Verwendung von ω-Aminosäuren wie ω-Aminocapronsäure als Alkalisierungsmittel ist möglich.

Erfolgt die Ausbildung der eigentlichen Haarfarben im Rahmen eines oxidativen Prozesses, so können übliche Oxidationsmittel, wie insbesondere Wasserstoffperoxid oder dessen Anlagerungsprodukte an Harnstoff, Melamin oder Natriumborat verwendet werden. Die Oxidation mit Luftsauerstoff als einzigem Oxidationsmittel kann allerdings bevorzugt sein. Weiterhin ist es möglich, die Oxidation mit Hilfe von Enzymen durchzuführen, wobei die Enzyme sowohl zur Erzeugung von oxidierenden Per-Verbindungen eingesetzt werden als auch zur Verstärkung der Wirkung einer geringen Menge vorhandener Oxidationsmittel, oder auch Enzyme die Elektronen aus geeigneten Entwicklerkomponenten (Reduktionsmittel) auf Luftsauerstoff übertragen. Bevorzugt sind dabei Oxidasen wie Tyrosinase, Ascorbatoxidase und Laccase aber auch Glucoseoxidase, Uricase oder Pyruvatoxidase. Weiterhin sei das Vorgehen genannt, die Wirkung geringer Mengen (z. B. 1% und weniger, bezogen auf das gesamte Mittel) Wasserstoffperoxid durch Peroxidasen zu verstärken.

Zweckmäßigerweise wird die Zubereitung des Oxidationsmittels dann unmittelbar vor dem Färben der Haare mit der Zubereitung mit den Farbstoffvorprodukten vermischt. Das dabei entstehende gebrauchsfertige Haarfärbepräparat sollte bevorzugt einen pH-Wert im Bereich von 6 bis 10 aufweisen. Besonders bevorzugt ist die Anwendung der Haarfärbemittel in einem schwach alkalischen Milieu. Die Anwendungstemperaturen können in einem Bereich zwischen 15 und 40 °C, bevorzugt bei der Temperatur der Kopfhaut, liegen. Nach einer Einwirkungszeit von ca. 5 bis 45, insbesondere 15 bis 30, Minuten wird das Haarfärbemittel durch Ausspülen von dem zu färbenden Haar entfernt. Das Nachwaschen mit einem Shampoo entfällt, wenn ein stark tensidhaltiger Träger, z. B. ein Färbeshampoo, verwendet wurde.

Insbesondere bei schwer färbbarem Haar kann die Zubereitung mit den Farbstoffvorprodukten ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert. Bei dieser Ausführungsform wird gemäß einer ersten Variante, bei der das vorherige Aufbringen der Farbstoffvorprodukte eine bessere Penetration in das Haar bewirken soll, das entsprechende Mittel auf einen pH-Wert von etwa 4 bis 7 eingestellt. Gemäß einer zweiten Variante wird zunächst eine Luftoxidation angestrebt, wobei das aufgebrachte Mittel bevorzugt einen pH-Wert von 7 bis 10 aufweist. Bei der anschließenden beschleunigten Nachoxidation kann die Verwendung von sauer eingestellten Peroxidisulfat-Lösungen als Oxidationsmittel bevorzugt sein.

Weiterhin kann die Ausbildung der Färbung dadurch unterstützt und gesteigert werden, daß dem Mittel bestimmte Metallionen zugesetzt werden. Solche Metallionen sind beispielsweise Zn²⁺, Cu²⁺, Fe²⁺, Fe³⁺, Mn²⁺, Mn⁴⁺, Li⁺, Mg²⁺, Ca²⁺ und Al³⁺. Besonders geeignet sind dabei Zn²⁺, Cu²⁺ und Mn²⁺. Die Metallionen können prinzipiell in der Form eines beliebigen, physiologisch verträglichen Salzes eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflußt werden.

Weitere fakultative Inhaltsstoffe der erfindungsgemäßen Mittel sind
- nichtionische Polymere wie beispielsweise Vinylpyrrolidon/Vinylacrylat-Copolymere, Polyvinylpyrrolidon und Vinylpyrrolidon/Vinylacetat-Copolymere,
- anionische Polymere, wie Polyacryl- und Polymethacrylsäuren in Form ihrer ihrer Copolymere mit Acrylsäure- und Methacrylsäureestern und -amiden, Polyoxycarbonsäuren, wie Polyketo- und Polyaldehydocarbonsäuren und deren Salze, sowie Polymere und Copolymere der Crotonsäure mit Estern und Amiden der Acryl- und der Methacrylsäure, wie Vinylacetat-Crotonsäure- und Vinylacetat-Vinylpropionat-Crotonsäure-Copolymere,
- Strukturanten wie Glucose und Maleinsäure,
- haarkonditionierende Verbindungen wie Phospholipide, beispielsweise Sojalecithin, Ei-Lecithin und Kephaline,
- Parfümöle, insbesondere solche mit der Duftnote einer Frucht, wie beispielsweise von Apfel, Birne, Erdbeere, Pfirsich, Aprikose, Ananas, Banane, Kirsche, Kiwi, Mango, Kokos, Mandel, Grapefruit, Maracuja, Mandarine und Melone, oder der Duftnote eines Genußmittels, wie beispielsweise von Tabak, Cola, Kaugummi, Guarana, Schokolade, Kakao, Vanille, Sarsaparilla, Pfefferminze und Rum.
   Dimethylisosorbid und Cyclodextrine,
- Lösungsvermittler, wie Ethanol, Isopropanol, Ethylenglykol, Propylenglykol, Glycerin, Diethylenglykol und ethoxylierte Triglyceride,
- Farbstoffe,
- Antischuppenwirkstoffe wie Climbazol, Piroctone Olamine und Zink Omadine,
- Wirkstoffe wie Bisabolol, Allantoin, Panthenol, Niacinmid, Tocopherol und Pflanzenextrakte,
- Lichtschutzmittel,
- Konsistenzgeber wie Zuckerester, Polyolester oder Polyolalkylether,
- Fette und Wachse, wie Walrat, Bienenwachs, Montanwachs, Paraffine, Ester, Glyceride und Fettalkohole,
- Fettsäurealkanolamide,
- Komplexbildner wie EDTA, NTA, β-Alanindiessigsäure und Phosphonsäuren,
- Quell- und Penetrationsstoffe wie PCA, Glycerin, Propylenglykolmonoethylether, Carbonate, Hydrogencarbonate, Guanidine, Harnstoffe sowie primäre, sekundäre und tertiäre Phosphate,
- Trübungsmittel wie Latex oder Styrol/Acrylamid-Copolymere,
- Perlglanzmittel wie Ethylenglykolmono- und -distearat oder PEG-3-distearat,
- Weißpigmente
- Reduktionsmittel wie z.B. Thioglykolsäure und deren Derivate, Thiomilchsäure, Cysteamin, Thioäpfelsäure und α-Mercaptoethansulfonsäure,
- Oxidationsmittel wie Wasserstoffperoxid, Kaliumbromat und Natriumbromat,
- Treibmittel wie Propan-Butan-Gemische, N₂O, Dimethylether, CO₂ und Luft sowie
- Antioxidantien,
- Bitterstoffe, wie beispielsweise Denatonium Benzoate,
- Konservierungsmittel.

Ebenfalls Gegenstand der Erfindung ist die Verwendung eines Mittels nach einem der Ansprüche 1 bis 16 zur Behandlung der Haare und/oder der Haut.

Die folgenden Beispiele sollen die Erfindung näher erläutern.

| **Rohstoff** | **1** | **2** | **3** | **4** | **5** | **6** | **7** |
|---|---|---|---|---|---|---|---|
| Ceraphyl® RMT | 0,5 | 1 | 1,5 | 1 | 1 | 0,5 | 3 |
| Texapon® N70 | 7 | | | 8 | | 10 | |
| Texapon® K14 S | | 7 | | | 4 | | 3 |
| Rewoteric® AM2 C NM | 3 | | 4 | | 2 | | 2 |
| Tego Betain® F50 | | 4 | 6 | 2 | 2 | 4 | |
| Eucarol® AGE-ET | 2 | | 4 | 3 | 2 | 4 | 2 |
| Protelan® AGL 95 | | 3 | | | 2 | | |
| Lamesoft® PO65 | 2 | | 3 | 5 | | 5 | |
| Varisoft® PATC | | | | | 1,2 | | |
| Merquat® 550 | | | 1 | | | | 0,5 |
| Jaguar® Excel | 0,1 | | | | | | |
| Polymer® JR400 | | 0,15 | | | | | |
| Cetiol® HE | | | 1 | 1 | 0,5 | | |
| Dow Corning® DC190 | | | | | | 1 | |
| Nexbase® 2004 | | | 0,5 | | | | |
| Hydriertes Ricinusöl (nanoisierte Rohstoffqualität) | | | | 0,5 | | | |
| D-Panthenol | 0,5 | | 1 | | | 2 | |
| Vitamin B3 | | 0,2 | | | | | |
| Herbasol-Extract Aloe | 1 | | | 0,5 | 0,4 | 0,5 | |
| Gluadin® W20 | 1 | | | | | 2 | 0,5 |
| Gluadin® WQ | | 1,5 | 1 | | | | |
| Cremophor® RH 455 | | | 0,5 | | 0,5 | 0,4 | 0,2 |
| Euperlan® PK 3000 | 2 | 4 | | 2 | | 3 | |
| Glutamate® DOE 120 | | 1 | | 1 | 0,4 | | |
| Antil® 141 L | 1 | | 0,5 | | 0,4 | 1 | 0,5 |
| Parfum | 0,3 | 0,2 | 0,5 | 0,4 | 0,7 | 1 | 0,3 |
| Natriumsalicylat | | 0,4 | 0,15 | 0,2 | | 0,5 | |
| Natriumbenzoat | 0,35 | | 0,15 | 0,3 | | | 0,4 |
| Natriumchlorid | q.s. | q.s. | q.s. | q.s. | q.s. | q.s. | q.s. |
| Zitronensäure | q.s. | q.s. | q.s. | q.s. | q.s. | q.s. | q.s. |
| Wasser | ad 100 | ad 100 | ad 100 | ad 100 | ad 100 | ad 100 | ad 100 |
| Viskosität / mPa s* | 4000 | 3500 | 5000 | 6000 | 6000 | 7000 | 3500 |
| pH-Wert | 5 | 5,5 | 4,8 | 5,2 | 5 | 5,5 | 4,8 |
| **1-3:** Shampoo, **4, 5:** Duschbad, **6:** Schaumbad, **7:** Flüssigseifes | | | | | | | |

| | | | | | | | |
|---|---|---|---|---|---|---|---|
| *: Haake VT 550-Rotationsviskosimeter (MV2-Drehkörper/Meßbecher; Scherrate: 7,2 l/s; 20°C. | | | | | | | |

### Bezeichnungen der Handelsprodukte nach INCI:

Ceraphyl® RMT: Castoryl Maleate
Texapon® N70: Sodium Laureth Sulfate, ca. 71% Aktivsubstanz (AS)
Texapon® K14 S: Sodium Myreth Sulfate, 70% AS
Rewoteric® AM2 C NM: Disodium Cocoamphodiacetate, 39% AS
Tego Betain® F50: Cocoamidopropyl Betaine, ca. 36% AS
Eucarol® AGE-ET: Sodium Cocopolyglucose Tartrate, 30% AS
Protelan® AGL 95: Sodium Lauroyl Glutamate, 33% AS
Lamesoft® PO65: Coco-Glucoside, Glyceryl Oleate, 65% AS
Varisoft® PATC: Palmitamidopropyltrimonium Chloride, ca. 59% AS
Merquat® 550: Polyquaternium-7, ca. 8,5% AS
Jaguar® Excel: Guar Hydroxypropyltrimonium Chloride
Polymer® JR400: Polyquaternium-10
Cetiol® HE: PEG-7 Glyceryl Cocoate
Dow Corning® DC190: Dimethicone Copolyol
Nexbase® 2004: Hydrogenated Polydecene
Vitamin B3: Niacinamide
Herbasol-Extract Aloe: Aloe Barbadensis
Gluadin® W20: Hydrolized Wheat Protein, 20% AS
Gluadin® WQ: Lauryldimonium Hydroxypropyl Hydrolized Wheat Protein, ca.33% AS
Cremophor® RH 455: PEG-40 Hydrogenated Castor Oil, Propylene Glycol, 90% AS
Euperlan® PK 3000: Glycol Distearate, Laureth-4, Cocoamidopropyl Betaine
Glutamate® DOE 120: PEG-120 Methyl Glucose Dioleate
Antil® 141 L: PEG-55 Propylene Glycol Oleate, Propylene Glycol, 40% AS

## Patentansprüche

1. Wäßriges kosmetisches Mittel zur Behandlung der Haare und/oder der Haut, **dadurch gekennzeichnet, daß** es neben üblichen kosmetischen Bestandteilen mindestens einen Wirkstoff (W) enthält, der ein Addukt einer Glyceridkomponente (G), ausgewählt aus Mono-, Di- oder Triglyceriden, die als Säurekomponente mindestens eine einfach oder mehrfach ungesättigte Hydroxycarbonsäure mit 10-30 Kohlenstoffatomen aufweisen, mit einem cyclischen Carbonsäureanhydrid (CA) darstellt.

2. Mittel nach Anspruch 1, **dadurch gekennzeichnet, daß** die Glyceridkomponente (G) ein Triglycerid ist.

3. Mittel nach Anspruch 1 oder 2, **dadurch gekennzeichnet, daß** die Hydroxycarbonsäure Ricinolsäure ist.

4. Mittel nach einem der Ansprüche 1 bis 3, **dadurch gekennzeichnet, daß** die Glyceridkomponente (G) das Triglycerid der Ricinolsäure ist.

5. Mittel nach einem der Ansprüche 1 bis 4, **dadurch gekennzeichnet, daß** das cyclische Carbonsäureanhydrid (CA) ausgewählt ist aus Bernsteinsäureanhydrid und Maleinsäureanhydrid.

6. Mittel nach einem der Ansprüche 1 bis 5, **dadurch gekennzeichnet, daß** das cyclische Carbonsäureanhydrid (CA) Maleinsäureanhydrid ist.

7. Mittel nach einem der Ansprüche 1 bis 6, **dadurch gekennzeichnet, daß** es weiterhin eine Vitaminkomponente enthält, ausgewählt aus der Gruppe, die die Vitamine, Pro-Vitamine und Vitaminvorstufen der Gruppen A, B, C, F und H sowie deren Derivate bilden.

8. Mittel nach Anspruch 7, **dadurch gekennzeichnet, daß** das Vitamin ausgewählt ist aus den Vitaminen der B-Gruppe, deren Vorstufen und deren Derivaten.

9. Mittel nach einem der Ansprüche 1 bis 8, **dadurch gekennzeichnet, daß** es ein Verdickungsmittel, ausgewählt aus der Gruppe, die alkoxylierte Fettalkohole, Zuckerderivate, gegebenenfalls vernetzte Polymere der Acrylsäure und der Methacrylsäure, alkoxylierte Methylglucosidester, alkoxylierte Propylenglykolester, Fettsäurepartialglyceridpolyglykolester und Fettsäurepartialglyceridpolyglykolether und deren Gemische umfaßt, enthält.

10. Mittel nach einem der Ansprüche 1 bis 9, **dadurch gekennzeichnet**, das es ein kationisches oder pseudokationisches Tensid enthält.

11. Mittel nach einem der Ansprüche 1 bis 10, **dadurch gekennzeichnet, daß** es ein Ampho-Polymer oder ein kationisches Polymer, ausgewählt aus der Gruppe, die kationische Cellulose-Derivate, kationische Guarderivate, kationische Polymere und Copolymere des Dimethyldiallylammoniumchlorids sowie kationische Copolymere des Vinylimidazoliummethochlorids umfaßt.

12. Mittel nach einem der Ansprüche 1 bis 11, **dadurch gekennzeichnet, daß** es ein anionisches Tensid, ausgewählt aus C₆-C₂₀-Acylglutamaten, Ethercarbonsäuresalzen sowie Estern von Hydroxydi- und -tricarbonsäuren mit polyhydroxylierten Verbindungen, enthält.

13. Mittel nach einem der Ansprüche 1 bis 12, **dadurch gekennzeichnet, daß** es ein Silikon enthält.

14. Mittel nach einem der Ansprüche 1 bis 13, **dadurch gekennzeichnet, daß** es einen Ölkörper, ausgewählt aus flüssigen Paraffinölen, Isoparaffinölen, synthetischen Kohlenwasserstoffen, Di-n-alkylethern, Esterölen, Kohlensäureestern mit Fettalkoholen, alkoxylierten Monoglyceriden sowie Dimeren, Telomeren und Polymeren von Olefinen enthält.

15. Mittel nach einem der Ansprüche 1 bis 14, **dadurch gekennzeichnet, daß** es ein Proteinhydrolysat oder dessen Derivat enthält.

16. Mittel nach einem der Ansprüche 1 bis 15, **dadurch gekennzeichnet, daß** es einen Feststoff, insbesondere einen Fettstoff, in nanopartikulärer Form enthält.

17. Verwendung eines Mittels nach einem der Ansprüche 1 bis 16 zur Behandlung der Haare und/oder der Haut.
